Europäisches Patentamt

European Patent Office

Office européen des brevets

(19)

(11) Numéro de publication: **0 582 494 B1**

(12) **FASCICULE DE BREVET EUROPEEN**

(45) Date de publication du fascicule du brevet: **29.11.95** (51) Int. Cl.6: **C07C 17/38**, C07C 19/08

(21) Numéro de dépôt: **93401639.5**

(22) Date de dépôt: **25.06.93**

(54) **Procédé de purification du 1,1,1,2-tetrafluoroéthane.**

(30) Priorité: **05.08.92 FR 9209700**

(43) Date de publication de la demande:
**09.02.94 Bulletin 94/06**

(45) Mention de la délivrance du brevet:
**29.11.95 Bulletin 95/48**

(84) Etats contractants désignés:
**BE DE ES FR GB GR IT NL**

(56) Documents cités:
**FR-A- 2 381 006**

(73) Titulaire: **ELF ATOCHEM S.A.**
**4 & 8, Cours Michelet**
**La Défense 10**
**F-92800 Puteaux (FR)**

(72) Inventeur: **Cheminal, Bernard**
**"La Rivière"-Orliénas**
**F-69530 Brignais (FR)**
Inventeur: **Lantz, André**
**Domaine de la Hêtraie**
**F-69390 Vernaison (FR)**

(74) Mandataire: **Leboulenger, Jean**
**Elf Atochem S.A.**
**Département Propriété Industrielle, Cedex 42**
**- La Défense 10**
**F-92091 Paris la Défense (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

**Description**

La présente invention concerne le domaine des hydrocarbures fluorés et a plus particulièrement pour objet la purification du 1,1,1,2-tétrafluoroéthane.

Ce composé, connu dans le métier sous la désignation F134a, est notamment destiné à remplacer le dichlorodifluorométhane (F12) actuellement utilisé comme fluide frigorifique, mais suspecté de contribuer à l'affaiblissement de la couche d'ozone strastosphérique. Pour ce faire, le F134a doit satisfaire à des normes de qualité relativement à la présence d'impuretés a priori toxiques comme les oléfines chlorofluorées. Parmi celles-ci figure notamment le 1-chloro-2,2-difluoroéthylène (F1122) qui, vu son point d'ébullition (-17,7 °C), s'avère très difficile à éliminer complètement du F134a (pEb. = -26,5 °C) par simple distillation, surtout sous pression.

Or, l'une des synthèses industrielle du F134a consiste en une fluoraton catalytique en phase gazeuse du 1-chloro-2,2,2-trifluoroéthane (F133a) qui sous-produit toujours des quantités variables de F1122 par réaction parasite de déshydrofluoration.

Pour résoudre ce problème, différentes techniques ont déjà été proposées. Ainsi, dans le brevet US 4 158 675 est décrit un procédé de traitement en ligne consistant à faire réagir les gaz issus de la réaction principale :

$$F133a + HF \rightleftharpoons F134a + HCl$$

dans un second réacteur maintenu à une température plus basse que celle de la réaction principale. A partir d'un mélange gazeux dont la teneur en F1122, ramenée aux composés organiques, est de 5300 vpm (volume par million), le traitement en ligne à 160 °C conduit à un taux de 7 vpm en F1122. L'inconvénient majeur de ce procédé réside dans la nécessité d'avoir à traiter un débit de gaz important et donc d'aboutir à un volume réactionnel élevé, ce qui entraîne un investissement et un coût d'entretien prohibitifs. D'autre part, la durée de vie du catalyseur (oxyde de chrome massique) n'est pas mentionnée et, pour compenser la perte d'activité catalytique, il peut s'avérer nécessaire d'augmenter progressivement la température, ce qui peut avoir entre autres pour conséquence immédiate une rétrogradation partielle du F134a en F133a par réaction sur HCl. De plus, la présence d'HCl dans les gaz risque également d'entraîner un problème de corrosion.

La demande de brevet EP 0 467 531 décrit un procédé de distillation des mélanges azéotropiques HF/F134a/F1122. La complexité de ce procédé et la grande consommation d'énergie qu'il nécessite ne le rendent pas particulièrement attractif.

Une autre technique, décrite dans la demande de brevet EP 0 446 869, consiste à réaliser la synthèse du F134a à partir de trichloréthylène et d'HF, en phase gazeuse, dans deux réacteurs disposés en série. Dans le premier on transforme à haute température le F133a en F134a accompagné de F1122 ; le flux sortant de ce premier réacteur, additionné de trichloréthylène, traverse un second réacteur catalytique à plus basse température pour convertir le trichloréthylène et le F1122 en F133a. L'inconvénient majeur d'un tel procédé réside dans la faible productivité du second réacteur, limitée par l'évacuation des calories produites par la réaction, d'une part, et par l'obligation de travailler avec un temps de contact élevé pour atteindre des taux très faibles de F1122, d'autre part. Un autre inconvénient de cette technique réside dans le risque de rétrogradation du F134a en F133a, en présence d'HCl dès que la température s'élève trop. Cette obligation de maîtriser parfaitement la température conduit alors à un risque d'apparition de F1122 non transformé puisque la réaction :

$$F133a \rightleftharpoons HF + F1122$$

équilibrée, dépend non seulement des rapports molaires HF/F1122 et F133a/F1122, mais aussi de la température et de la pression. A cet égard, on peut citer le brevet EP 36123 relatif à l'addition catalytique d'HF sur les oléfines (chloro)fluorées, notamment le F1122 pur (exemple 4) dont la fluoration à température contrôlée (120 à 131 °C) laisse subsister du F1122 non transformé en sortie de réacteur.

Comme autres moyens d'élimination des oléfines, on peut citer :

- L'hydrogénation catalytique (demande de brevet WO 90/08750) : cette méthode nécessite des catalyseurs coûteux (métaux précieux) et conduit à des composés saturés hydrogénés, différents du F133a, à séparer ultérieurement ; d'autre part, l'hydrogène entraîne toujours un peu de F134a par tension de vapeur.
- L'adsorption physique sur un mélange d'oxydes de manganèse et de cuivre (hopcalite) : l'inconvénient majeur de ce procédé, décrit dans la demande de brevet EP 370 688, réside dans la nécessité

2

de régénérer régulièrement après usage cet adsorbant solide; il en résulte des pertes de F134a par adsorption plus ou moins sélective et une concentration importante en oléfine au moment du cycle de régénération.

- La distillation extractive (demande de brevet EP 472 391) des mélanges F134a/F1122 au moyen de solvants appropriés (trichloréthylène, perchloréthylène, etc...) : le principal inconvénient de cette technique réside dans la complexité de l'appareillage (colonnes à distiller supplémentaires pour recycler le solvant purifié), l'incidence élevée du coût énergétique (vaporisations successives) et le rendement plus ou moins efficace du traitement.

Pour éviter les inconvénients des techniques précitées, la présente invention propose un moyen particulièrement efficace et économique pour purifier un F134a brut contenant des impuretés insaturées.

Le procédé selon l'invention consiste à traiter en phase gazeuse, en l'absence d'acide chlorhydrique, un mélange gazeux de F134a brut et d'HF à une température comprise entre 200 et 380°C et sous une pression allant de la pression atmosphérique jusqu'à 2,5 MPa, en présence d'un catalyseur de fluoration, le rapport molaire HF/F134a étant compris entre 0,05 et 0,5.

Durant ce traitement, l'acide fluorhydrique s'additionne sur les oléfines (chloro)fluorées, telles que le F1122, le 1,2,2-trifluoroéthylène (F1123) et le 1,1,1,4,4,4-hexafluorobutène ($CF_3CH = CHCF_3$), et les transforme en composés saturés faciles à séparer et/ou à recycler par distillation.

Dans un F134a brut, le taux d'impuretés oléfiniques peut varier entre 100 et 10000 ppm (0,01 à 1 %) par rapport au F134a et est le plus souvent compris entre 500 et 5000 ppm (0,05 à 0,5 %). A côté des oléfines (chloro)fluorées, le F134a brut peut également contenir des quantités variables d'autres composés comme, par exemple, le F133a (0 à 7 %), le 1,1,1-trifluoroéthane (F143a) et le pentafluoroéthane (F125). La présence de ces impuretés saturées ne nuit nullement à l'efficacité du procédé selon l'invention.

Le traitement catalytique en phase gazeuse selon l'invention est avantageusement réalisé à une température comprise entre 225 et 325°C et, de préférence, sous une pression comprise entre la pression atmosphérique et 1,5 MPa.

Le temps de contact peut varier entre 5 et 100 secondes, mais on préfère un temps de contact compris entre 25 et 75 secondes.

Comme indiqué précédemment, le rapport molaire HF/F134a peut varier entre 0,05 et 0,5. On préfère cependant opérer avec un rapport molaire HF/F134a compris entre 0,125 et 0,200 et, plus particulièrement, un rapport molaire proche de celui correspondant à l'azéotrope HF-134a (0,15).

A l'issue du traitement selon l'invention, le flux gazeux ne contient plus, ou seulement des traces, d'impuretés oléfiniques et peut alors être soumis aux opérations conventionnelles (décantation, distillation, lavage à l'eau, neutralisation, ...) pour séparer l'HF non transformé et les composés saturés autres que le F134a.

Les catalyseurs de fluoration à utiliser pour la mise en oeuvre du procédé selon l'invention peuvent être des catalyseurs massiques ou des catalyseurs supportés, le support stable dans le milieu réactionnel étant, par exemple, un charbon actif, le fluorure d'aluminium ou le phosphate d'aluminium.

Parmi les catalyseurs massiques, on peut citer plus particulièrement l'oxyde de chrome préparé selon l'une quelconque des méthodes connues de l'homme du métier (procédé sol-gel, précipitation de l'hydroxyde à partir des sels de chrome, réduction de l'anhydride chromique, etc...). Les dérivés de métaux tels que nickel, fer, manganèse, cobalt, peuvent également convenir seuls ou en association avec le chrome, sous forme de catalyseurs massiques, mais aussi sous forme de catalyseurs supportés.

Les catalyseurs supportés peuvent être employés sous forme de billes, d'extrudés, de pastilles ou même, si l'on opère en lit fixe, sous forme de morceaux. Pour les catalyseurs massiques, la forme de pastilles ou de billes est généralement préférée. Lorsqu'on opère en lit fluide, on préfère utiliser un catalyseur sous forme de billes ou d'extrudés.

Comme exemples non limitatifs de catalyseurs, on peut mentionner :

- les microbilles d'oxyde de chrome obtenues par le procédé sol-gel comme décrit dans le brevet FR 2 501 062,
- les catalyseurs d'oxyde de chrome déposé sur charbon actif (brevet US 4 474 895), sur phosphate d'aluminium (brevet EP 55 958) ou sur fluorure d'aluminium (brevets US 4 579 974 et 4 579 976).
- les catalyseurs mixtes d'oxyde de chrome et de fluorure de nickel déposés sur fluorure d'aluminium (demande de brevet EP 0 486 333).

Les brevets précités dont le contenu est incorporé ici par référence décrivent largement le mode de préparation de ces catalyseurs, mais aussi leur mode d'activation, c'est-à-dire de transformation préalable du catalyseur en espèces actives stables par fluoration au moyen d'HF gazeux dilué par des composés inertes (azote) ou non (air ou 1,1,2-trichloro-1,2,2-trifluoroéthane). Durant cette activation, les oxydes métalliques servant de matière active (par exemple l'oxyde de chrome) ou de support (par exemple

3

l'alumine) peuvent être partiellement ou complètement transformés en fluorures correspondants.

Les exemples suivants illustrent l'invention sans la limiter.

# EXEMPLE 1

Dans un réacteur tubulaire en Inconel 600 de 28 mm de diamètre intérieur et d'un volume de 200 ml, on place 50 ml d'un catalyseur constitué de microbilles d'oxyde de chrome massique, préparé comme décrit à l'exemple 3 du brevet FR 2 501 062. On alimente ensuite ce réacteur catalytique fonctionnant en lit fixe avec un mélange, à l'état gazeux, constitué de F134a brut et d'HF dans des proportions telles que le rapport molaire HF/F134a soit égal à 0,25.

Le F134a brut a la composition pondérale suivante :

. F134a : 97 %
. F1122 : 0,08 %
. F133a : 0,45 %
. F125 + F143a : 2 %

La température du réacteur est fixée à 225°C et sa pression absolue à 1,5 MPa. Le débit (d) d'alimentation du mélange F134a brut + HF varie de telle sorte que le temps de contact (t) évolue entre 11 et 55 secondes, d et t étant liés par la relation :

$$t = \frac{15 \times 3600 \times V \times 273}{22,4 \times d \times (T+273)}$$

où

$t =$ temps de contact en secondes
$d =$ débit en moles/heure
$V =$ volume de catalyseur en vrac, exprimé en litres
$T =$ température du réacteur en degré Celsius

Un échantillon gazeux, débarrassé de l'HF en excès, est analysé par CPV en sortie de réacteur pour suivre l'évolution du taux de F1122 dans les produits organiques. Le rendement (R) d'élimination du F1122 est défini par la relation :

$$R \ (\text{en } \%) = 100 \times \frac{C_i - C_f}{C_i}$$

où $C_i$ est la concentration initiale en F1122 dans les réactifs organiques alimentés au réacteur et $C_f$ la concentration finale en F1122 dans les produits organiques en sortie de réacteur, ces concentrations étant exprimées en volume par million (vpm).

Le tableau I suivant rassemble les résultats obtenus sur la même charge catalytique en fonction du temps de contact et de l'âge du catalyseur.

## TABLEAU I

| ESSAI | CONDITIONS OPERATOIRES | | | RESULTATS | |
|---|---|---|---|---|---|
| N° | Temps de contact (sec.) | Durée de l'essai (heures) | Age du catalyseur (heures)* | Cf (vpm) | R (%) |
| 1-A | 22 | 5 | 16,5 | < 20 | > 97,6 |
| 1-B | 30 | 5 | 21,5 | < 5 | > 99,4 |
| 1-C | 52 | 5 | 26,5 | < 5 | > 99,4 |
| 1-D | 12 | 3 | 29,5 | env.80 | > 88,9 |
| 1-E | 32 | 24 | 53,5 | < 5 | > 99,3 |
| 1-F | 30 | 2 | 55,5 | < 10 | > 98,6 |
| 1-G | 30 | 30 | 83,5 | < 10 | > 98,6 |
| 1-H | 30 | 71 | 124,5 | < 15 | > 97,8 |
| 1-I(**) | 29 | 6 | 130,5 | < 5 | > 99,3 |
| 1-J(**) | 11 | 3 | 133,5 | env.30 | 96 |

(*) A la fin de l'essai
(**) Essais réalisés à 250°C

L'examen de ces résultats montre qu'on obtient à 225°C une élimination quasi-totale du F1122 avec un temps de contact d'environ 30 secondes et que le rendement d'élimination du F1122 est encore correct lorsque l'âge cumulé du catalyseur atteint 124,5 heures (Essais 1-E à 1-H).

Une élévation de la température de 225 à 250°C (essais 1-I et 1-J), sur la même charge de catalyseur, permet de retrouver un taux d'élimination quasitotal pour un temps de contact d'environ 30 secondes (essai 1-I). Pour un temps de contact de 11 secondes (essai 1-J), le rendement d'élimination chute à 96 %.

## EXEMPLE 2

Dans le même réacteur qu'à l'exemple 1, on introduit 100 ml d'un catalyseur à base de fluorure de nickel et d'oxyde de chrome déposés sur fluorure d'aluminium. Les caractéristiques physico-chimiques de ce catalyseur, préparé comme décrit dans la demande de brevet EP 0 486 333 et activé en lit fixe par un mélange azote/HF, sont les suivantes :

| - Composition chimique (pondérale) : | |
|---|---|
| . fluor | 58,6 % |
| . aluminium | 25,9 % |
| . nickel | 6,4 % |
| . chrome | 6,0 % |

| - Propriétés physiques : | |
|---|---|
| . densité apparente (en vrac) | 0,85 g/cm$^3$ |
| . surface BET | 23 m$^2$/g |
| . volume des pores d'un rayon compris entre 40Å et 63 µm | 0,4 cm$^3$/g |
| . surface des pores d'un rayon supérieur à 40Å | 23 m$^2$/g |

Après une ultime activation "in situ" du catalyseur à l'aide d'un mélange gazeux HF/F133a (rapport molaire : 0,4) entre 25 et 250°C, on alimente le réacteur avec un mélange gazeux constitué d'HF et de F134a brut dans des proportions telles que le rapport molaire HF/F134a soit égal à 0,18, c'est-à-dire proche de la composition azéotropique.

Le F134a brut utilisé a la composition molaire suivante :

| . F134a | 95,9 % |
|---|---|
| . F1122 | 0,24 % |
| . F133a | 3,8 % |
| . F125 | 0,02 % |
| . F143a | 0,04 % |

En travaillant sous une pression absolue de 1,0 MPa, avec un temps de contact d'environ 50 secondes et à différentes températures (250, 300 et 350°C), on a obtenu les résultats rassemblés dans le tableau II suivant.

## TABLEAU II

| ESSAI | CONDITIONS OPERATOIRES | | | | RESULTATS | |
|---|---|---|---|---|---|---|
| N° | Tempé-rature (°C) | Temps de contact (sec.) | Durée de l'essai (h) | Age du cataly-seur (h)* | Cf (vpm) | R (%) |
| 2-A | 250 | 50,8 | 6 | 6 | < 5 | 99,8 |
| 2-B | " " | 45,3 | 18 | 24 | < 5 | 99,8 |
| 2-C | 300 | 50,7 | 7 | 31 | < 5 | 99,8 |
| 2-D | " " | 50,0 | 13,5 | 44,5 | < 5 | 99,8 |
| 2-E | " " | 52,1 | 9,5 | 54 | < 5 | 99,8 |
| 2-F | " " | 50,4 | 17,5 | 71,5 | < 5 | 99,8 |
| 2-G | 350 | 54,2 | 8 | 79,5 | 11 | 99,5 |
| 2-H | " " | 47,8 | 15 | 94,5 | 13 | 99,4 |
| 2-I | 250 | 52,4 | 7,5 | 102 | < 5 | 99,8 |
| 2-J | " " | 50,8 | 19 | 121 | < 5 | 99,8 |

(*) A la fin de l'essai

Les résultats obtenus sont très performants, même à haute température sur la même charge de catalyseur. Les essais 2-I et 2-J confirment parfaitement l'activité du catalyseur et sa résistance aux

changements de température.

## EXEMPLE 3

On utilise un réacteur tubulaire en Inconel 600 de 38 mm de diamètre intérieur et d'un volume utile de 400 ml, dans lequel on place 200 ml d'un catalyseur commercial d'oxyde de chrome massique en pastilles de 4,8 x 4,8 mm, préalablement activé en lit fixe à l'aide d'un mélange azote/HF.

Les caractéristiques physico-chimiques de ce catalyseur, après activation, sont les suivantes :

| - Composition chimique (pondérale) : | |
|---|---|
| . fluor | 20,0 % |
| . chrome | 56,3 % |
| . carbone | 3,5 % |
| . oxygène | 20,2 % |

| - Propriétés physiques : | |
|---|---|
| . densité apparente (en vrac) | 1,21 g/cm$^3$ |
| . surface BET | 124 m$^2$/g |
| . volume des pores d'un rayon compris entre 40Å et 63 $\mu$m | 0,14 cm$^3$/g |
| . surface des pores d'un rayon supérieur à 40Å | 42,3 m$^2$/g |

Après une ultime activation "in situ" du catalyseur, à 350°C sous pression atmosphérique, à l'aide d'un mélange gazeux HF/F133a (rapport molaire : 4) et avec un temps de contact de 4 secondes, on alimente le réacteur avec un mélange gazeux constitué d'HF et de F134a brut dans des proportions telles que le rapport molaire HF/F134a soit égal à 0,18, c'est-à-dire proche de la composition azéotropique.

Le F134a brut utilisé a la composition molaire suivante :

| . F134a | 95,6 % |
|---|---|
| . F1122 | 0,19 % |
| . F133a | 3,0 % |
| . F125 | 0,05 % |
| . F143a | 0,05 % |

En travaillant sous une pression absolue de 1,0 MPa, avec un temps de contact variant entre 25 et 100 secondes et à différentes températures (225 et 250°C, puis 200°C et à nouveau 225°C), on a obtenu les résultats rassemblés dans le tableau III suivant.

EP 0 582 494 B1

## TABLEAU III

| ESSAI | CONDITIONS OPERATOIRES | | | | RESULTATS | |
|---|---|---|---|---|---|---|
| N° | Tempé-rature (°C) | Temps de contact (sec.) | Durée de l'essai (h) | Age du cataly-seur (h)* | Cf (vpm) | R (%) |
| 3-A | 225 " " | 49,5 " " | 5,5 16,5 5,5 | 5,5 22 27,5 | < 5 " " | >99,9 " " |
| 3-B | 250 " " | 49,8 " " | 5,1 16,4 2 | 32,6 49 51 | < 5 " " | >99,9 " " |
| 3-C | 250 " | 97,5 " | 6,25 16,75 | 57,25 74 | < 5 " | >99,9 " |
| 3-D | 200 " | 50,4 " | 5 18 | 79 97 | 60 130 | 96,8 93,1 |
| 3-E | 200 " | 26,4 " | 8 17 | 105 122 | 400 425 | 78,7 77,3 |
| 3-F | 225 | 49,0 | 23 | 145 | 30 | 98,4 |

(*) A la fin de l'essai

L'examen des résultats montre qu'une température de 200°C, surtout avec un temps de contact d'environ 26 secondes (essais 3-D et 3-E), ne permet pas d'aboutir à une élimination quasi-complète du F1122.

L'essai de contrôle 3-F, réalisé à nouveau à 225°C et avec un temps de contact de 49 secondes, laisse apparaître 30 vpm de F1122 non transformé (au lieu de moins de 5 vpm pour l'essai 3-A), ce qui indique une légère baisse d'activité du catalyseur après 145 heures de fonctionnement continu.

## EXEMPLE 4

Dans le même réacteur qu'à l'exemple 1, on introduit 100 ml d'un catalyseur au chrome sur charbon actif (3 mm) présentant, après activation, les caractéristiques physico-chimiques suivantes :

| | |
|---|---|
| . teneur en chrome | 20,3 % en poids |
| . teneur en fluor | 13,6 % en poids |
| . surface BET | 204 $m^2/g$ |
| . surface des pores d'un rayon supérieur à 40Å | 15,4 $m^2/g$ |
| . volume des pores d'un rayon compris entre 40Å et 63 $\mu$m | 0,43 $cm^3/g$ |

On alimente ensuite ce réacteur catalytique fonctionnant en lit fixe avec un mélange gazeux ayant la composition molaire suivante :

| . F134a | 81,9 % |
|---------|--------|
| . F1122 | 0,11 % |
| . F133a | 2,6 % |
| . HF | 15,4 % |

et on travaille à 250°C sous une pression absolue de 1MPa et avec un temps de contact d'environ 70 secondes.

En opérant dans ces conditions, la concentration (Cf) en F1122 dans les produits organiques en sortie de réacteur est inférieure à 2 vpm, soit un rendement d'élimination d'au moins 99,9 %. Ces performances sont parfaitement stables ; elles ont été obtenues pendant 740 heures de fonctionnement.

**Revendications**

1. Procédé de purification d'un 1,1,1,2-tétrafluoroéthane (F134a) brut contenant des impuretés insaturées, caractérisé en ce que l'on traite en phase gazeuse, en l'absence d'acide chlorhydrique, un mélange gazeux de 1,1,1,2-tétrafluoroéthane brut et d'acide fluorhydrique à une température comprise entre 200 et 380°C et sous une pression allant de la pression atmosphérique jusqu'à 2,5 MPa, en présence d'un catalyseur de fluoration, le rapport molaire HF/F134a étant compris entre 0,05 et 0,5.

2. Procédé selon la revendication 1, dans lequel le traitement catalytique en phase gazeuse est effectué à une température comprise entre 225 et 325°C.

3. Procédé selon la revendication 1 ou 2, dans lequel on opère sous une pression comprise entre la pression atmosphérique et 1,5 MPa.

4. Procédé selon l'une des revendications 1 à 3, dans lequel le temps de contact est compris entre 5 et 100 secondes, de préférence entre 25 et 75 secondes.

5. Procédé selon l'une des revendications 1 à 4, dans lequel le rapport molaire HF/F134a est compris entre 0,125 et 0,200.

6. Procédé selon l'une des revendications 1 à 5, dans lequel le catalyseur de fluoration est un catalyseur massique ou supporté à base de chrome, nickel, fer, manganèse et/ou cobalt.

7. Procédé selon la revendication 6, dans lequel le catalyseur est l'oxyde de chrome sous forme de microbilles ou supporté sur charbon actif, phosphate d'aluminium ou fluorure d'aluminium, ou un mélange d'oxyde de chrome et de fluorure de nickel déposés sur un support de fluorure d'aluminium.

**Claims**

1. Process for the purification of a crude 1,1,1,2-tetrafluoroethane (F134a) containing unsaturated impurities, characterised in that a gaseous mixture of crude 1,1,1,2-tetrafluoroethane and hydrofluoric acid is treated in the gas phase, in the absence of hydrochloric acid, at a temperature of between 200 and 380°C and under a pressure ranging from atmospheric pressure to 2.5 MPa, in the presence of a fluorination catalyst, the HF/F134a molar ratio being between 0.05 and 0.5.

2. Process according to Claim 1, in which the catalytic treatment in the gas phase is carried out at a temperature of between 225 and 325°C.

3. Process according to Claim 1 or 2, in which the operation is carried out under a pressure between atmospheric pressure and 1.5 MPa.

4. Process according to one of Claims 1 to 3, in which the contact time is between 5 and 100 seconds, preferably between 25 and 75 seconds.

5. Process according to one of Claims 1 to 4, in which the HF/F134a molar ratio is between 0.125 and 0.200.

**6.** Process according to one of Claims 1 to 5, in which the fluorination catalyst is a bulk or supported catalyst based on chromium, nickel, iron, manganese and/or cobalt.

**7.** Process according to Claim 6, in which the catalyst is chromium oxide in the form of microbeads or supported on active charcoal, aluminium phosphate or aluminium fluoride, or a mixture of chromium oxide and nickel fluoride deposited on a support of aluminium fluoride.

**Patentansprüche**

**1.** Verfahren zur Reinigung von technischem 1,1,1,2-Tetrafluorethan (F134a), das ungesättigte Verunreinigungen enthält, dadurch gekennzeichnet, daß man in der Gasphase in Abwesenheit von Chlorwasserstoff ein gasförmiges Gemisch von technischem 1,1,1,2-Tetrafluorethan und Fluorwasserstoff bei einer Temperatur von 200 bis 380 °C und einem Druck zwischen Atmosphärendruck und 2,5 MPa in Anwesenheit eines Fluorierungskatalysators behandelt, wobei das Molverhältnis HF/F134a 0,05 bis 0,5 beträgt.

**2.** Verfahren nach Anspruch 1, bei dem die katalytische Behandlung in der Gasphase bei einer Temperatur von 225 bis 325 °C durchgeführt wird.

**3.** Verfahren nach Anspruch 1 oder 2, bei dem man unter einem Druck zwischen Atmosphärendruck und 1,5 MPa arbeitet.

**4.** Verfahren nach einem der Ansprüche 1 bis 3, bei dem die Kontaktzeit zwischen 5 und 100 Sekunden, vorzugsweise zwischen 25 und 75 Sekunden, beträgt.

**5.** Verfahren nach einem der Ansprüche 1 bis 4, bei dem das Molverhältnis HF/F134a 0,125 bis 0,200 beträgt.

**6.** Verfahren nach einem der Ansprüche 1 bis 5, bei dem der Fluorierungskatalysator ein Katalysator auf Basis von Chrom, Nickel, Eisen, Mangan und/oder Kobalt in Masse oder auf ein Trägermaterial aufgebracht ist.

**7.** Verfahren nach Anspruch 6, bei dem der Katalysator ein Chromoxid ist, das in Form von Mikrokügelchen oder auf Aktivkohle, Aluminiumphosphat oder Aluminiumfluorid aufgebracht vorliegt, oder als eine Mischung aus Chromoxid und Nickelfluorid vorliegt, die auf Aluminiumfluorid als Trägermaterial aufgebracht ist.